(19) Europäisches Patentamt
European Patent Office
Office européen des brevets

(11) EP 0 897 716 B1

(12) **EUROPÄISCHE PATENTSCHRIFT**

(45) Veröffentlichungstag und Bekanntmachung des Hinweises auf die Patenterteilung:
**14.09.2005 Patentblatt 2005/37**

(51) Int Cl.7: **A61K 7/42**, C08G 77/04

(21) Anmeldenummer: **98115411.5**

(22) Anmeldetag: **17.08.1998**

(54) **Wirkstoffkombinationen aus einer oder mehreren grenzflächenaktiven Substanzen und oligomeren oder polymeren UV-Filtersubstanzen mit periodisch sich wiederholenden Si-O-Gruppen**

Combination of active agents containing one or more surface active agents and oligomeric or polymeric U.V. filtering substances with periodically appearing Si-O groups

Combinaisons d'agents actifs contenant un ou plusieurs agents tensio-actifs et des substances U.V. filtrantes oligomériques ou polymériques avec une répétition périodique de groupements Si-O

(84) Benannte Vertragsstaaten:
**DE ES FR GB IT SE**

(30) Priorität: **19.08.1997 DE 19735899**

(43) Veröffentlichungstag der Anmeldung:
**24.02.1999 Patentblatt 1999/08**

(73) Patentinhaber: **Beiersdorf Aktiengesellschaft**
**20245 Hamburg (DE)**

(72) Erfinder: **Gers-Barlag, Heinrich, Dr.**
**25495 Kummerfeld (DE)**

(56) Entgegenhaltungen:
**EP-A- 0 383 655**
**EP-A- 0 679 389**
**WO-A-93/04665**
**WO-A-93/04666**
**WO-A-98/35649**
**FR-A- 2 758 982**

- **DATABASE WPI Section Ch, Week 197920 Derwent Publications Ltd., London, GB; Class A25, AN 1979-37652B XP002122682 & JP 54 043212 A (LION FAT & OIL CO LTD), 5. April 1979 (1979-04-05)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 897 716 B1

## Beschreibung

Die vorliegende Erfindung betrifft Lichtschutzformulierungen, insbesondere kosmetische und dermatologische Lichtschutzmittel.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Im allgemeinen ist das Lichtabsorptionsverhalten von Lichtschutzfiltersubstanzen sehr gut bekannt und dokumentiert, zumal in den meisten Industrieländem Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Der Lichtschutzfaktor (LSF, oft auch, dem englischen Sprachgebrauch angepaßt, SPF genannt) gibt an, um wieviel länger die mit dem Lichtschutzmittel geschützte Haut bestrahlt werden kann, bis die gleiche Erythemreaktion auftritt wie bei der ungeschützten Haut (also zehnmal solang gegenüber ungeschützter Haut bei LSF = 10).

Jedenfalls erwartet der Verbraucher zum einen - nicht zuletzt wegen der ins Licht der Öffentlichkeit gerückten Diskussion über das sogenannte "Ozonloch" zum einen zuverlässige Angaben des Herstellers zum Lichtschutzfaktor, zum anderen geht eine Tendenz des Verbrauchers zu höheren und hohen Lichtschutzfaktoren.

Kosmetische oder dermatologische UV-Filtersubstanzen liegen in Lichtschutzformulierungen üblicherweise in gelöster Form vor. Gemäß Formulierungen des Standes der Technik war es erheblich erschwert, solche Substanzen als Festkörper derart homogen verteilt in den entsprechenden Formulierungen einzusetzen, daß, ohne einen erheblichen Wirksamkeitsverlust einigermaßen befriedigende Produkte erhältlich waren.

Da Lichtschutzfiltersubstanzen in der Regel kostspielig sind, und da manche Lichtschutzfiltersubstanzen zudem schwierig in höheren Konzentrationen in kosmetische oder dermatologische Zubereitungen einzuarbeiten sind, war es Aufgabe der Erfindung, auf einfache und preiswerte Weise zu Zubereitungen zu gelangen, welche bei ungewöhnlich niedrigen Konzentrationen an herkömmlichen Lichtschutzfiltersubstanzen dennoch akzeptable oder sogar hohe LSF-Werte erreichen.

Die Schriften EP-A-0 383 655 und WO 93/04665 konnten nicht den Weg zur vorliegenden Erfindung weisen.

In den Schriften EP-A-392 883, WO 92/20690 und WO 93/04665 werden vorteilhafte UV-Filtersubstanzen beschrieben, welche sich dadurch auszeichnen, daß chromophore Gruppen mit guten UV-Absorptionseigenschaften an ein Grundgerüst gebunden sind, welches als Organosiloxan charakterisiert werden kann.

Die Einarbeitung dieser UV-Filtersubstanzen in kosmetische oder pharmazeutische Zubereitungen und/oder

die Erzielung hoher Lichtschutzfaktoren mit diesen UV-Filtersubstanzen war jedoch schwierig.

Es war indes überraschend, daß die Verwendung von grenzflächenaktiven Substanzen der der allgemeinen Formel

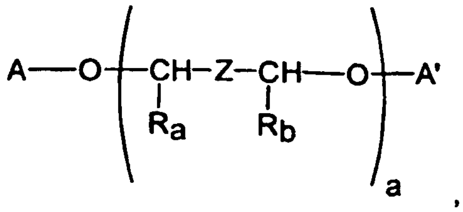

wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

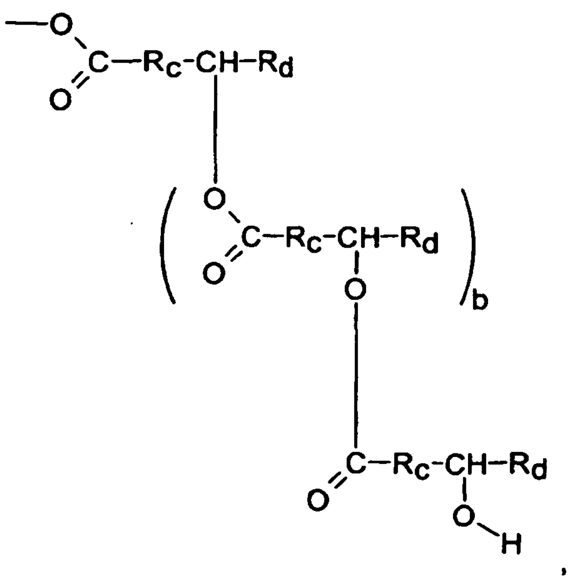

wobei $R_c$ gewählt wird aus der Gruppe der verzweigten und unverzweigten Alkylgruppen mit 1 bis 20 Kohlenstoffatomen und $R_d$ gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- Z eine Einfachbindung oder die Gruppe

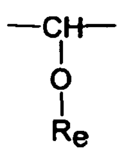

- darstellt,
- $R_a$ und $R_b$ unabhängig voneinander aus der Gruppe H, Methyl gewählt werden,
- $R_e$ gewählt wird aus der Gruppe H, sowie der verzweigten und unverzweigten, gesättigten und ungesättigten Alkyl- und Acylreste mit 1 - 20 Kohlenstoffatomen,

zur Erhöhung des Lichtschutzfaktors kosmetischer oder dermatologischer Zubereitungen welche oligomere oder polymere UV-Filtersubstanzen mit periodisch sich wiederholenden Si-O- Gruppen enthalten, gewählt werden aus der Gruppe der Substanzen, die durch folgende allgemeine Strukturen beschrieben werden:

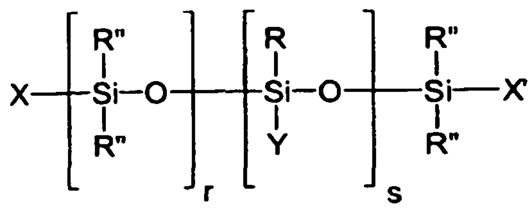

(Strukturschema I)

bzw.

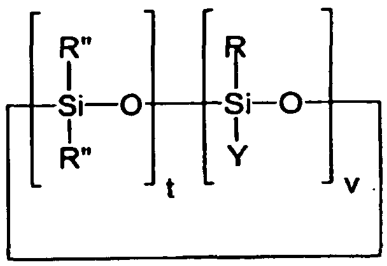

(Strukturschema II),

wobei

- die Reste R und R" Alkylreste mit 1 - 10 Kohlenstoffatomen bzw. Phenylreste repräsentieren.
- die Reste X, X' entweder ebenfalls organische Reste wie R und R" oder aber Reste wie Y, repräsentieren
- r Werte von 0 - 100 annimmt
- s Werte von 0 - 20 annimmt, wobei mindestens einer der Reste X, X' einen Rest Y bedeutet, wenn s = 0.
- t Werte von 0 - 10 annimmt,
- v Werte von 1 - 10 annimmt, wobei die Summe aus v und t größer oder gleich 3 ist.
- Y stellt die für die UV-Absorption verantwortlichen Chromophorreste dar und insbesondere vorteilhaft gewählt wird aus der Gruppe

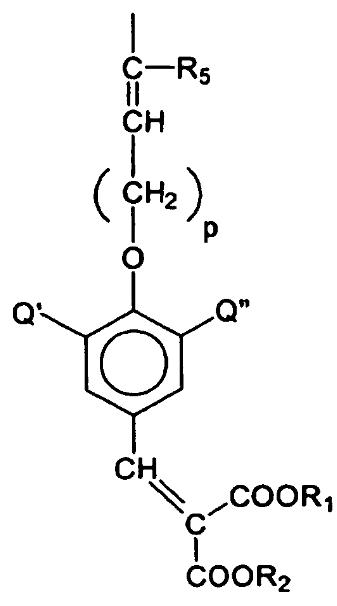

und/oder

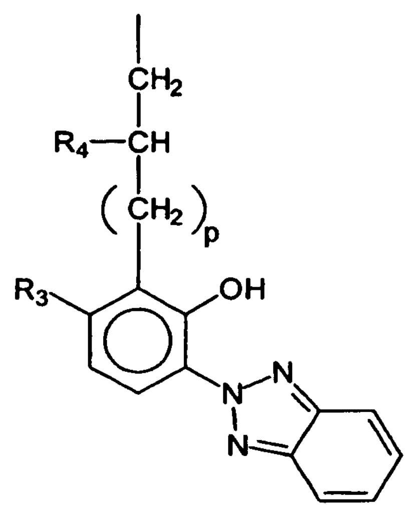

,

wobei $R_1$, $R_2$, unabhängig voneinander verzweigte oder unverzweigte Alkylgruppen von 1 - 10 Kohlenstoffatomen darstellen, und wobei $R_3$, $R_4$, $R_5$ unabhängig voneinander Wasserstoffatome bzw. verzweigte oder unverzweigte Alkylgruppen von 1 - 10 Kohlenstoffatomen darstellen und p eine Zahl von 1 - 10 darstellt. Q' und/oder Q" können unabhängig voneinander darstellen: H, Hydroxygruppen, verzweigte oder unverzweigte Alkylgruppen von 1 - 10 Kohlenstoffatomen, verzweigte oder unverzweigte Alkoxygruppen mit 1 - 10 Kohlenstoffatomen.

die Nachteile des Standes der Technik beseitigen.

Die Strukturformel der grenzflächenaktiven Substanz oder Substanzen ist nicht so zu interpretieren, daß durch den Index a alle in der Klammer repräsentierten Reste $R_a$, $R_b$ bzw $R_e$ im gesamten Molekül jeweils gleich sein müssen. Vielmehr können diese Reste in jedem der a Fragmente

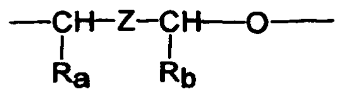

frei gewählt werden.

Ein Beispiel für besonders vorteilhaft im Sinne der vorliegenden Erfindung zu verwendende grenzflächenaktiven Substanzen ist das PEG-30 Dipolyhydroxystearat, welches von der Gesellschaft ICI Surfactants unter der Marke ARLACEL® P135 verkauft wird.

Erfindungsgemäß können die oligomeren oder polymeren UV-Filtersubstanzen mit periodisch sich wiederholenden Si-O- Gruppen vorteilhaft aus der Gruppe der UV-Filtersubstanzen gewählt werden, die in den Schriften EP-A-392 883, WO 92/20690 und WO 93/04665 UV-Filtersubstanzen beschrieben werden.

Grob schematisch können diese oligomeren oder polymeren UV-Filtersubstanzen, die im folgenden der Einfachheit halber auch "Polymerfilter" genannt werden sollen, durch folgende allgemeine Strukturen beschrieben werden:

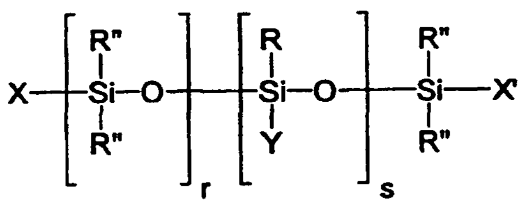

**(Strukturschema I)**

bzw.

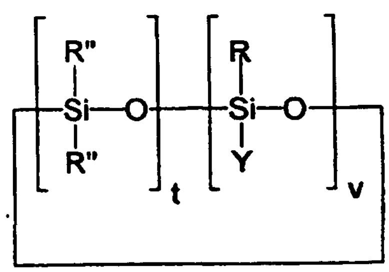

**(Strukturschema II)**

Dabei repräsentieren die Reste R und R" allgemeine organische Reste, die Reste X, X' und repräsentieren entweder ebenfalls organische Reste wie R und R" oder aber Reste wie Y, was weiter unten erläutert wird. r kann Werte von 0 - 100 annehmen, s kann Werte von 0 - 20 annehmen, wobei mindestens einer der Rest e X, X' einen Rest Y bedeutet, wenn s = 0. t kann Werte von 0 - 10 annehmen, v kann Werte von 1 - 10 annehmen, wobei die Summe aus v und t größer oder gleich 3 ist.

Y stellt die für die UV-Absorption verantwortlichen Chromophore dar. Insbesondere repräsentieren die Reste R und R" Alkylreste mit 1 - 10 Kohlenstoffatomen bzw. Phenylreste. Die Chromophorreste Y werden insbesondere vorteilhaft gewählt aus der Gruppe

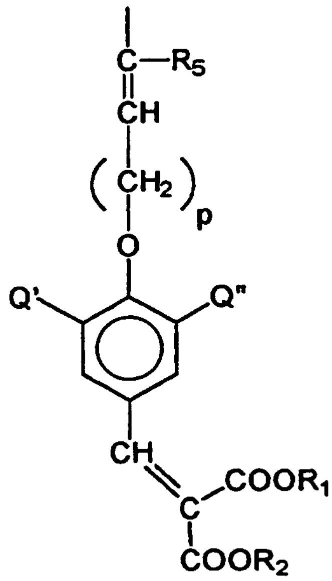

und/oder

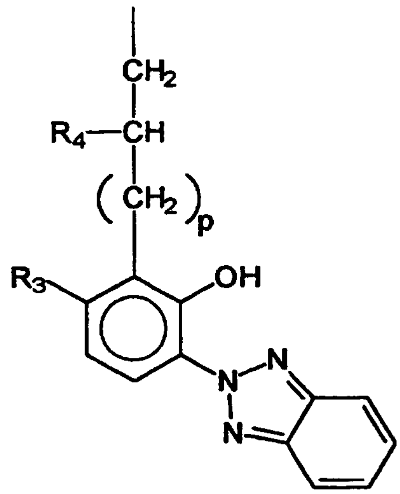

,

wobei $R_1$, $R_2$, unabhängig voneinander verzweigte oder unverzweigte Alkylgruppen von 1 - 10 Kohlenstoffatomen darstellen, und wobei $R_3$, $R_4$, $R_5$ unabhängig voneinander Wasserstoffatome bzw. verzweigte oder unverzweigte Alkylgruppen von 1 - 10 Kohlenstoffatomen darstellen und p eine Zahl von 1 - 10 darstellt. Q' und/oder Q" können unabhängig voneinander darstellen: H, Hydroxygruppen, verzweigte oder unverzweigte Alkylgruppen von 1 - 10 Kohlenstoffatomen, verzweigte oder unverzweigte Alkoxygruppen mit 1 - 10 Kohlenstoffatomen.

Ganz besonders vorteilhaft wird Y gewählt aus der Gruppe

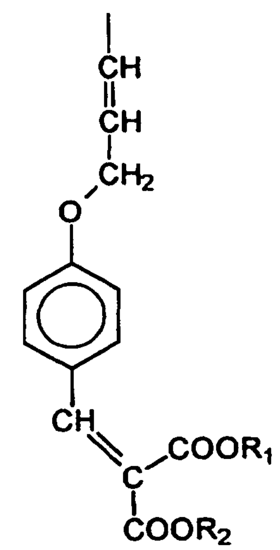

,

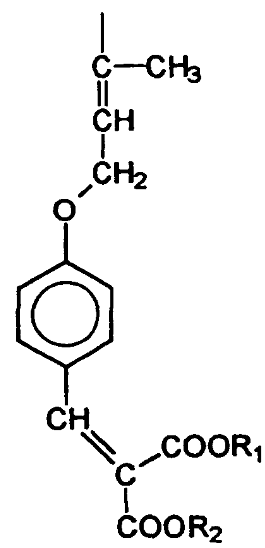

,

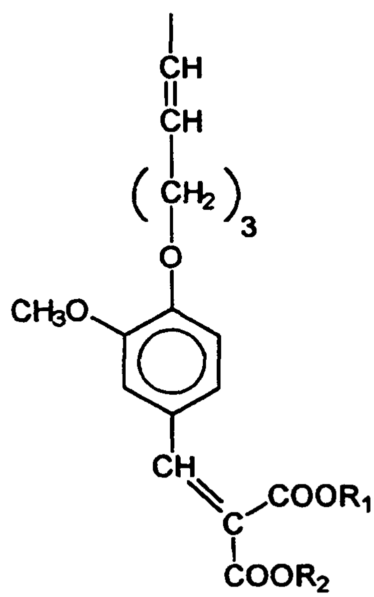

,

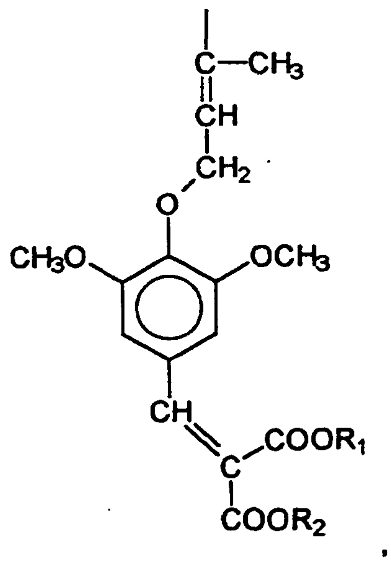

wobei $R_1$ und $R_2$ unabhängig voneinander Methyl oder Ethyl darstellen.

Bevorzugter erfindungsgemäß verwendeter Polymerfilter sind Substanzen, in welchen Y die Gruppe

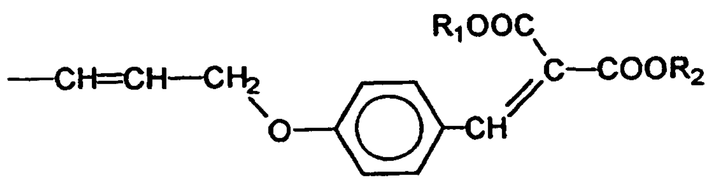

darstellt, wobei R bzw. R'' Methylgruppen darstellen, und in in welchen $R_1$ und/oder $R_2$ Ethyl- und/oder Methylgruppen darstellen und bei welcher das stöchiometrische Verhältnis von Si-O- Einheiten, welche Chromophorgruppen Y tragen, zu Si-O- Einheiten, welche keine Chromophorgruppen Y tragen, im Bereich von 1 : 20 bis 1 : 10, insbesondere etwa 1:15 liegt.

Besonders vorteilhafte Ausführungsformen der vorliegenden Erfindung werden aber auch erhalten, wenn als Y gewählt wird der Rest

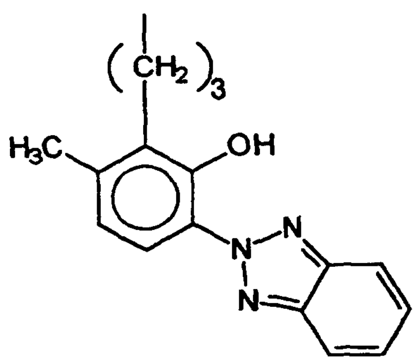

Die durch die Indices r, s, t, v gegebene Periodizität in den Polymerfiltern ist ebenfalls nicht so zu interpretieren, daß bei vorgegebenem Index alle Reste Y bzw. R bzw. R'' usw. identisch sein müssen.

Es war überraschend und für den Fachmann nicht vorauszusehen, daß die Verwendung von erfindungsgemäß verwendeten grenzflächenaktiven Substanzen zur Erhöhung des Lichtschutzfaktors kosmetischer oder dermatologischer Zubereitungen, welche erfindungsgemäß verwendete Polymerfilter enthalten, führen würde.

Weiterhin war überraschend und für den Fachmann nicht vorauszusehen, daß die Verwendung von erfindungsgemäß verwendeten grenzflächenaktiven Substanzen zur Verbesserung der Einarbeitbarkeit erfindungsgemäß verwendete Polymerfilter in kosmetische oder dermatologische Zubereitungen führen würde.

Die Gesamtmenge an erfindungsgemäß verwendeten grenzflächenaktiven Substanzen in den erfindungsgemäßen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an erfindungsgemäß verwendeten Polymerfiltern in den erfindungsgemäßen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Erfindungsgemäße kosmetische und dermatologische Zubereitungen enthalten vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Solche anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \text{ -> } n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Zubereitungen gemäß der Erfindung können auch röntgenamporphe Oxidpigmente enthalten. Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente erhältlich durch Flammenreaktion, beispielsweise dadurch, daß ein Metall- oder Halbmetallhalogenid mit Wasserstoff und Luft (oder reinem Sauerstoff) in einer Flamme umgesetzt wird.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe

enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder-monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxyd, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß wird der LSF durch die Verwendung von Polymerfiltern in kosmetischen oder dermatologischen Zubereitungen, enthaltend übliche UVA-Filter und/oder UVB-Filter in der Lipidphase und/oder in der wäßrigen Phase erheblich gesteigert.

Insbesondere wird der LSF durch die Verwendung von Polymefiltem in kosmetischen oder dermatologischen Zubereitungen, enthaltend Substanzen, die UV-Strahlung im UVB-Bereich absorbieren, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Solche UVB-Filter können öllöslich oder wasserlöslich sein. Vorteilhafte öllösliche UVB-Filtersubstanzen sind z.B.:

- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Vorteilhafte wasserlösliche UVB-Filtersubstanzen sind z.B.:

- Sulfonsäure-Derivate des 3-Benzylidencamphers, wie z.B. 4-(2-Oxo-3-bornylidenmethyl)benzolsulfonsäure,2-Methyl-5-(2-oxo-3-bomylidenmethyl)sulfonsäure und deren Salze.

Die Liste der genannten UVB-Filter, die zusätzlich im Sinne der vorliegenden Erfindung eingesetzt werden können, soll selbstverständlich nicht limitierend sein.

Insbesondere wird der LSF durch die Verwendung von Polymerfiltern in kosmetischen oder dermatologischen Zubereitungen, enthaltend Substanzen, die UV-Strahlung im UVA-Bereich absorbieren, wobei die Gesamtmenge solcher Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, erheblich gesteigert.

Die Gesamtmenge an wasserlöslichen UV-Filtersubstanzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Phenylbenzimidazol-5-sulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Hydroxy-4-methoxybenzophenon-5-sulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(2-Oxo-3-bornylidenmethyl)benzolsulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Methyl-5-(2-oxo-3-bornylidenmethyl)benzolsulfonsäur bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzol-1,4-di(2-oxo-3-bornylidenmethyl-10-Sulfonsäurebzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4,4',4''-(1,3,5-Triazin-2,4,6-triyltriimino)-tris-benzoesäure-tris(2-ethylhexylester) in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(tert.-Butyl)-4'-methoxydibenzoylmethan in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-Methylbenzylidencampher in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Ethylhexyl-p-methoxy-cinnamat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Noch eine weiterere erfindungsgemäß vorteilhaft zu verwendende zusätzliche Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

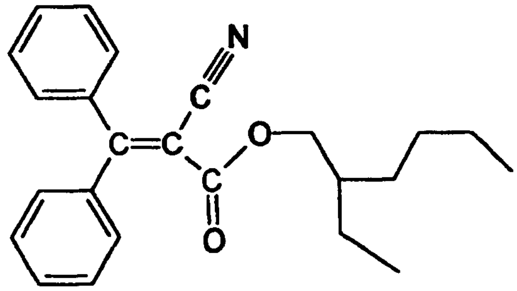

Die Gesamtmenge an Ethylhexyl-2-cyano-3,3-diphenylacrylat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 10,0 Gew.-% gewählt, bezogen auf das Gesamtgewicht der Zubereitungen.

Ferner kann gegebenenfalls von Vorteil sein, erfindungsgemäß weiteren UVA- und/oder UVB-Filtem in kosmetische oder dermatologisch Zubereitungen einzuarbeiten, beispielsweise bestimmten Salicylsäurederivaten wie 4-Isopropylbenzylsalicylat, 2-Ethylhexylsalicylat (=Octylsalicylat), Homomenthylsalicylat.

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Der LSF kosmetischer oder dermatologischer Zubereitungen, enthaltend beiliebige der vorab geschilderten UV-Filtersubstanzen, sei es als Einzelsubstanzen oder in beliebigen Gemischen untereinander, wobei die Gesamtmenge solcher Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, wird erfindungsgemäß durch die Verwendung von erfindungsgemäß eingesetzten Wirkstoffkombinationen erheblich gesteigert.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die Bezeichnung "Polymerfilter X" bedeutet in den Beispielen eine Substanz, in welcher Y die Gruppe

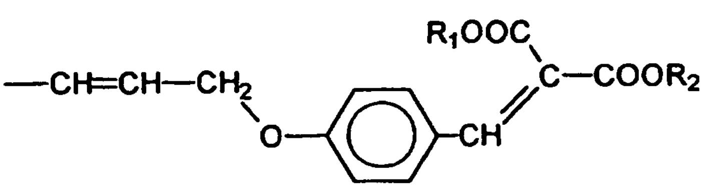

darstellt, in welcher R bzw. R" Methylgruppen darstellen, und in in welchen $R_1$ und $R_2$ Ethylgruppen darstellen und bei welcher das stöchiometrische Verhältnis von Si-O- Einheiten, welche Chromophorgruppen Y tragen, zu Si-O- Einheiten, welche keine Chromophorgruppen Y tragen, etwa 1:15 beträgt.

**<u>Beispiel 1</u>**

| Sonnenschutzcrème | |
|---|---|
| Arlacel® P135 | 5,00 |
| Softisan® 100 | 3,00 |
| Glycerin | 3,00 |
| Lunacera S | 0,50 |

(fortgesetzt)

| Sonnenschutzcrème | |
|---|---|
| Magnesiumsulfat | 0,70 |
| Mineralöl | 3,00 |
| Polymerfilter X | 10,00 |
| Caprylyl Ether | 8,00 |
| Uvinul® T150 | 3,00 |
| Cetearylisononanoat | 6,00 |
| Konservierungsmittel | q.s. |
| Parfum | q.s. |
| Wasser, demin. | ad 100,00 |

**Beispiel 2**

| Sonnenschutzlotion | |
|---|---|
| Arlacel®P135 | 5,00 |
| DC Q2-5200 | 1,20 |
| Magnesiumstearat | 0,05 |
| Buthylenglykol | 5,00 |
| Elfacos®C26 | 1,00 |
| Magnesiumsulfat | 0,50 |
| Isohexadecan | 7,00 |
| Capric/Caprylic Triglyceride | 5,00 |
| Cetearylisononanoat | 7,00 |
| Polymerfilter X | 3,00 |
| Octocrylen | 5,00 |
| ZnO | 2,50 |
| $TiO_2$ | 10,00 |
| Uvinul®T150 | 5,00 |
| Konservierungsmittel | q.s. |
| Parfum | q.s. |
| Wasser, demin. | ad 100,00 |

**Beispiel 3**

| Sonnenschutzlotion | |
|---|---|
| Arlacel® P135 | 3,00 |
| Magnesiumstearat | 0,05 |
| Butylenglycol | 5,00 |
| Elfacos®C26 | 1,00 |
| Magnesiumsulfat | 0,50 |
| Isohexadecan | 7,00 |
| Capric/Caprylic Triglyceride | 5,00 |
| Cetearylisononanoat | 7,00 |
| Polymerfilter X | 7,00 |
| Eusolex® 232 | 3,00 |
| Parsol®1789 | 2,00 |

(fortgesetzt)

| Sonnenschutzlotion | |
|---|---|
| Eusolex®6300 | 4,00 |
| Uvinul®T150 | 5,00 |
| Konservierungsmittel | q.s. |
| Parfum | q.s. |
| Wasser, demin. | ad 100,00 |

## Patentansprüche

**1.** Verwendung von von grenzflächenaktiven Substanzen der allgemeinen Formel

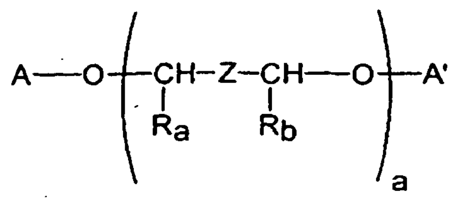

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

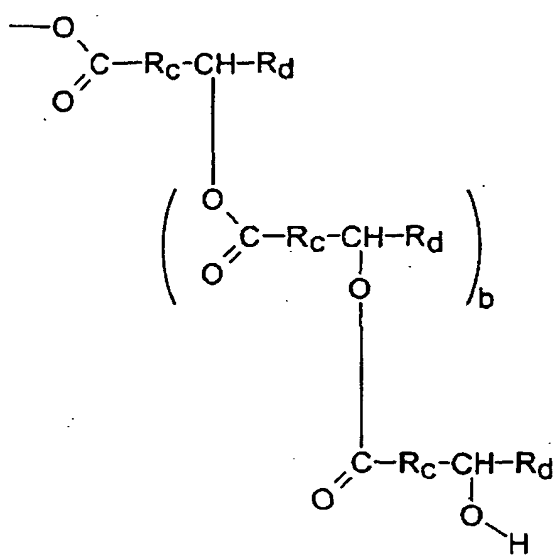

,

wobei $R_c$ gewählt wird aus der Gruppe der verzweigten und unverzweigten Alkylgruppen mit 1 bis 20 Kohlenstoffatomen und $R_d$ gewählt wird aus der Gruppe der verzweigten und unverzweigten Alkylengruppen mit 1 bis 20 Kohlenstoffatomen und b Zahlen von 0 bis 200 annehmen kann,
- a eine Zahl von 1 bis 100, vorzugsweise 2 bis 60, insbesondere 5 bis 40 darstellt,
- Z eine Einfachbindung oder die Gruppe

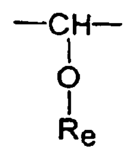

- darstellt,
- $R_a$ und $R_b$ unabhängig voneinander aus der Gruppe H, Methyl gewählt werden,
- $R_e$ gewählt wird aus der Gruppe H, sowie der verzweigten und unverzweigten, gesättigten und ungesättigten Alkyl- und Acylreste mit 1 - 20 Kohlenstoffatomen,

zur Erhöhung des Lichtschutzfaktors kosmetischer oder dermatologischer Zubereitungen, welche oligomere oder polymere UV-Filtersubstanzen mit periodisch sich wiederholenden Si-O- Gruppen enthalten, gewählt aus der Gruppe der Substanzen, die durch folgende allgemeine Strukturen beschrieben werden:

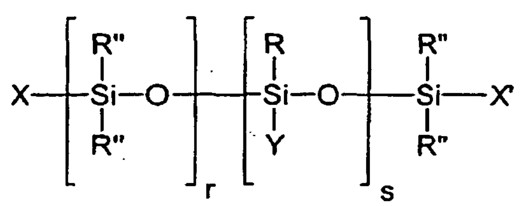

(Strukturschema I)

bzw.

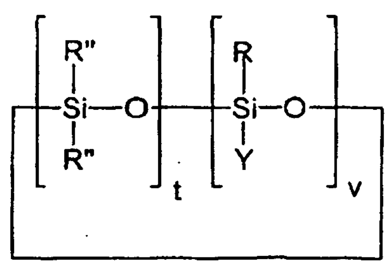

(Strukturschema II),

wobei

- die Reste R und R" Alkylreste mit 1 - 10 Kohlenstoffatomen bzw. Phenylreste repräsentieren.
- die Reste X, X' entweder ebenfalls organische Reste wie R und R" oder aber Reste wie Y, repräsentieren
- r Werte von 0 - 100 annimmt
- s Werte von 0 - 20 annimmt, wobei mindestens einer der Reste X, X' einen Rest Y bedeutet, wenn s = 0.
- t Werte von 0 - 10 annimmt,
- v Werte von 1 - 10 annimmt, wobei die Summe aus v und t größer oder gleich 3 ist.
- Y stellt die für die UV-Absorption verantwortlichen Chromophorreste dar und insbesondere vorteilhaft gewählt wird aus der Gruppe

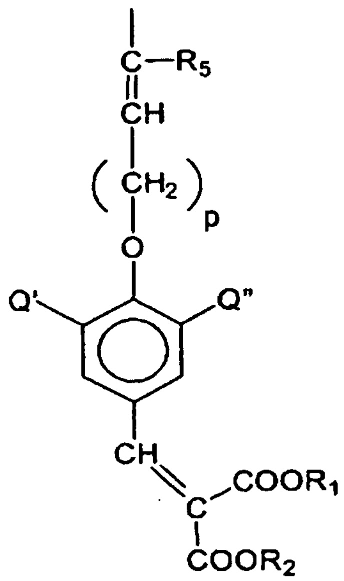

und/oder

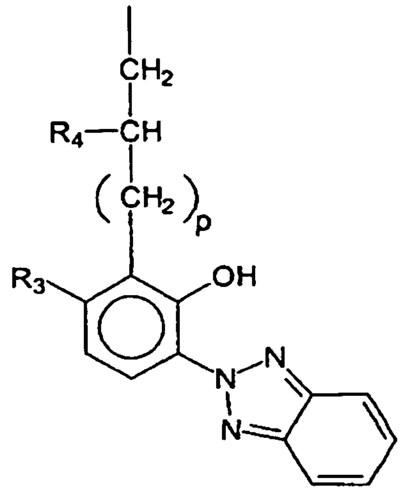

,

wobei $R_1$, $R_2$, unabhängig voneinander verzweigte oder unverzweigte Alkylgruppen von 1 - 10 Kohlenstoffatomen darstellen, und wobei $R_3$, $R_4$, $R_5$ unabhängig voneinander Wasserstoffatome bzw. verzweigte oder unverzweigte Alkylgruppen von 1 - 10 Kohlenstoffatomen darstellen und p eine Zahl von 1 - 10 darstellt. Q' und/oder Q" können unabhängig voneinander darstellen: H, Hydroxygruppen, verzweigte o der unverzweigte Alkylgruppen von 1 - 10 Kohlenstoffatomen, verzweigte oder unverzweigte Alkoxygruppen mit 1 - 10 Kohlenstoffatomen.

## Claims

**1.** Use of interface-active substances of the general formula

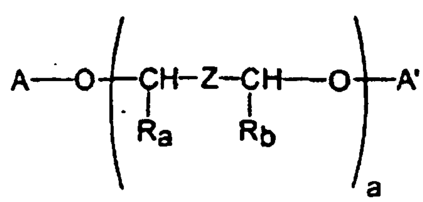

,

- where A and A' are identical or different organic radicals chosen from the group of branched and unbranched saturated and unsaturated alkyl and acyl radicals and hydroxyacyl radicals having 10-30 carbon atoms, and also from the group of hydroxyacyl groups joined together via ester functions, according to the scheme

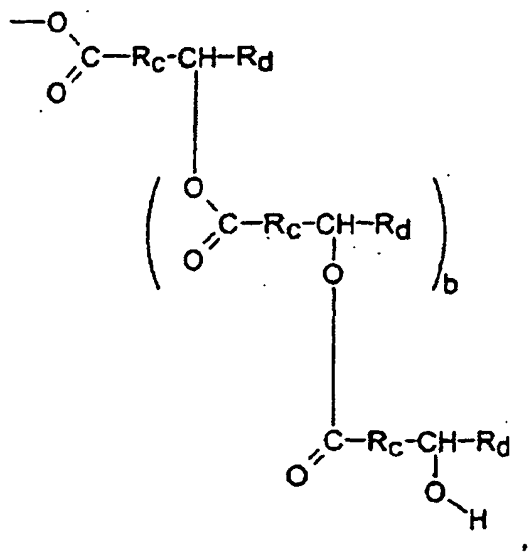

where $R_c$ is chosen from the group of branched and unbranched alkyl groups having 1 to 20 carbon atoms and $R_d$ is chosen from the group of branched and unbranched alkylene groups having 1 to 20 carbon atoms and b can assume numbers from 0 to 200,

- a is a number from 1 to 100, preferably 2 to 60, in particular 5 to 40,
- Z is a single bond or the group

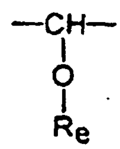

- $R_a$ and $R_b$, independently of one another, are chosen from the group consisting of H, methyl,
- $R_e$ is chosen from the group consisting of H, and branched and unbranched, saturated and unsaturated alkyl and acyl radicals having 120 carbon atoms,

for increasing the sun protection factor of cosmetic or dermatological preparations which comprise oligomeric or polymeric UV filter substances with periodically repeating Si-O groups, chosen from the group of substances which are described by the following general structures:

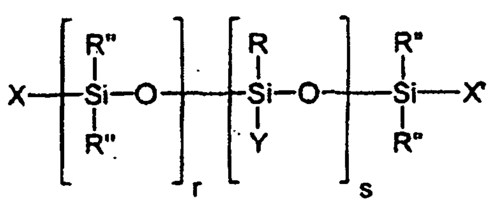

(Structure scheme I)

and

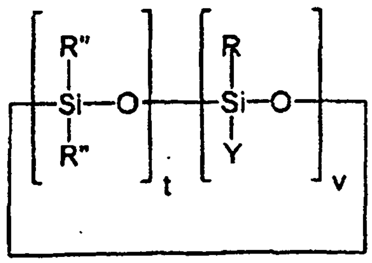

(Structure scheme II),

where

- the radicals R and R" represent alkyl radicals having 1-10 carbon atoms or phenyl radicals,
- the radicals X, X' either likewise represent organic radicals, such as R and R" or else radicals such as Y,
- r assumes values from 0-100
- s assumes values from 0-20, where at least one of the radicals X, X' is a radical Y if s = 0,
- t assumes values from 0-10,
- v assumes values from 1-10, where the sum of v and t is greater than or equal to 3,
- Y is the chromophore radicals responsible for UV absorption and is chosen particularly advantageously from the group

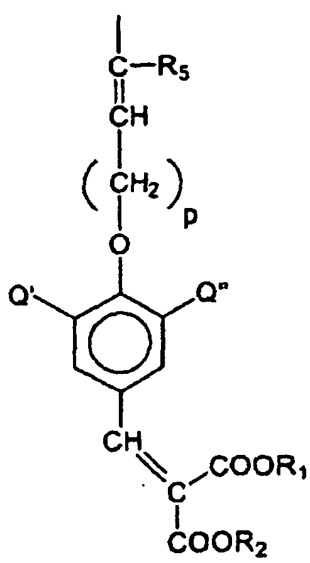

and/or

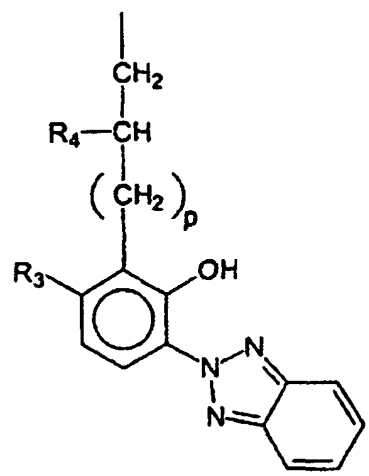

,

where $R_1$, $R_2$, independently of one another, are branched or unbranched alkyl groups of 1-10 carbon atoms, and where $R_3$, $R_4$, $R_5$, independently of one another, are hydrogen atoms or branched or unbranched alkyl groups of 1-10 carbon atoms and p is a number from 1-10. Q' and/or Q" may, independently of one another, be: H, hydroxyl groups, branched or unbranched alkyl groups of 1-10 carbon atoms, branched or unbranched alkoxy groups with 1-10 carbon atoms.

## Revendications

**1.** Utilisation de substances tensioactives de formule générale:

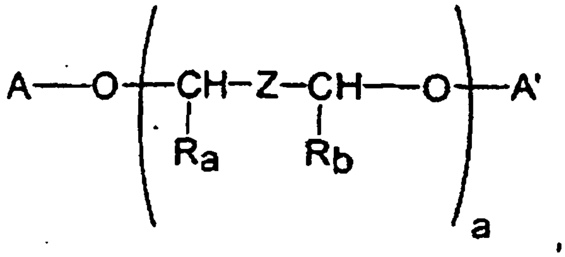

- dans laquelle A et A' représentent des radicaux organiques identiques ou différents sélectionnés dans l'ensemble des radicaux alkyle et acyle linéaires ou ramifiés, saturés ou non saturés, et des radicaux hydroxyacyle qui comptent de 10 à 30 atomes de carbone ou dans l'ensemble des groupes hydroxyalkyle liés les uns aux autres par des fonctions ester, selon le schéma:

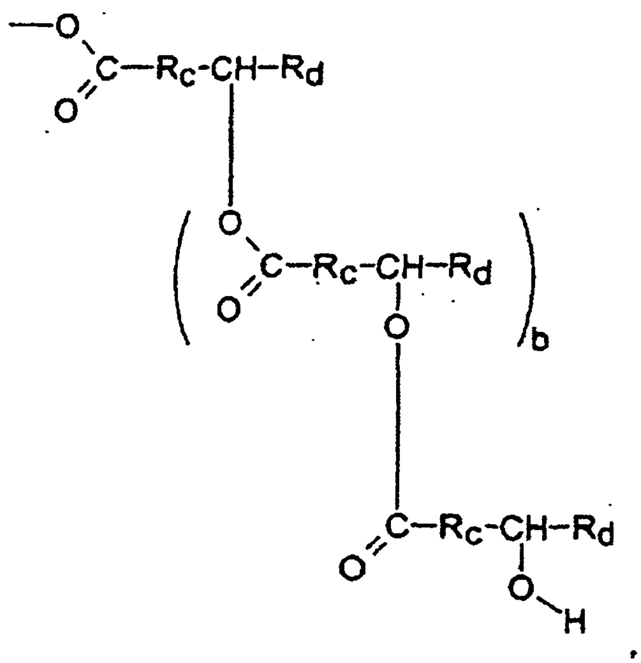

dans lequel $R_c$ est sélectionné dans l'ensemble des groupes alkyle linéaires ou ramifiés qui comptent de 1 à 20 atomes de carbone et $R_d$ est sélectionné dans l'ensemble des groupes alkylène linéaires ou ramifiés qui comptent de 1 à 20 atomes de carbone, b représentant un nombre qui peut prendre une valeur comprise entre 0 et 200,
- a représente un nombre de 1 à 100, de préférence de 2 à 60 et en particulier de 5 à 40,
- Z représente une liaison simple ou le groupe:

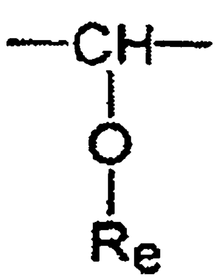

- $R_a$ et $R_b$ sont sélectionnés indépendamment l'un de l'autre dans le groupe constitué de H et du méthyle,
- $R_e$ est sélectionné dans l'ensemble constitué de H et des radicaux alkyle et acyle linéaires ou ramifiés, saturés ou non saturés qui comptent de 1 à 20 atomes de carbone,

pour augmenter l'indice de protection lumineuse de préparations cosmétiques ou dermatologiques qui contiennent des substances oligomères ou polymères de filtration des UV avec des groupes Si-O- qui se répètent périodiquement,
qui sont sélectionnées dans l'ensemble des substances qui sont décrites par les structures générales ci-dessous:

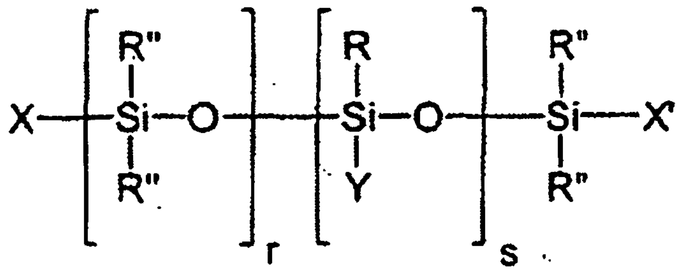

(schéma structurel I)

et

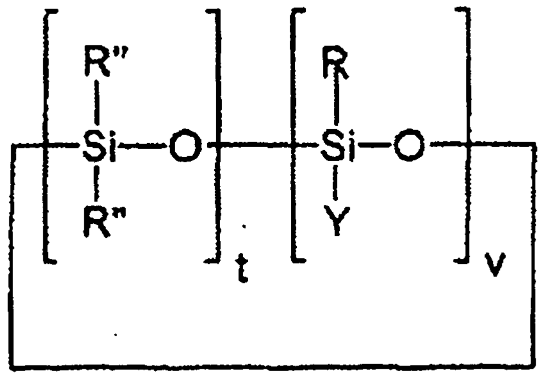

(schéma structurel II),

dans lesquels

- les radicaux R et R" représentent des radicaux alkyle qui comptent de 1 à 10 atomes de carbone ou des radicaux phényle,
- les radicaux X, X' représentent également des radicaux organiques tels que R et R" ou des radicaux tels que Y,
- r a une valeur comprise entre 0 et 100,
- s a une valeur comprise entre 0 et 20, au moins l'un des radicaux X, X' représentant un radical Y lorsque s = 0,
- t a une valeur comprise entre 0 et 10,
- v a une valeur comprise entre 1 et 10, la somme de v et de t étant supérieure ou égale à 3,
- Y représente le radical chromophore responsable de l'absorption des UV et est en particulier sélectionné avantageusement dans l'ensemble constitué de:

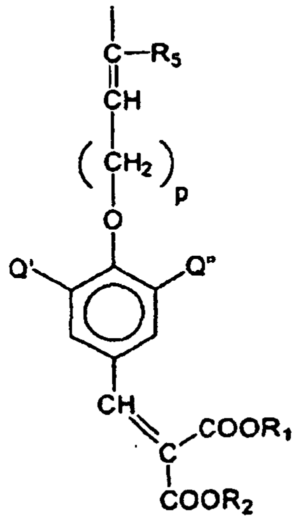

et/ou

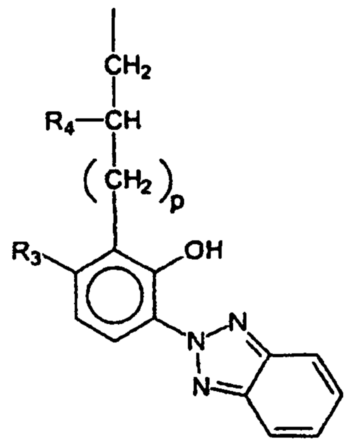

,

$R_1$, $R_2$ représentant indépendamment l'un de l'autre des groupes alkyle linéaires ou ramifiés qui comptent de 1 à 10 atomes de carbone, $R_3$, $R_4$, $R_5$ représentant indépendamment l'un de l'autre des atomes d'hydrogène ou des groupes alkyle linéaires ou ramifiés qui comptent de 1 à 10 atomes de carbone et p représente un nombre compris entre 1 et 10. Q' et/ou Q" peuvent représenter indépendamment l'un de l'autre H, des groupes hydroxyle, des groupes alkyle linéaires ou ramifiés qui comptent de 1 à 10 atomes de carbone ou des groupes alcoxy linéaires ou ramifiés qui comptent de 1 à 10 atomes de carbone.